(19) Europäisches Patentamt
European Patent Office
Office européen des brevets

(11) EP 4 678 622 A1

(12) EUROPEAN PATENT APPLICATION

(43) Date of publication:
**14.01.2026 Bulletin 2026/03**

(21) Application number: **24186979.1**

(22) Date of filing: **07.07.2024**

(51) International Patent Classification (IPC):
***C07C 29/14** (2006.01)* ***C07C 29/17** (2006.01)*
***C07C 45/69** (2006.01)* ***C07C 33/14** (2006.01)*
***C07C 31/137** (2006.01)* ***C07C 47/445** (2006.01)*

(52) Cooperative Patent Classification (CPC):
(C-Sets available)
**C07C 45/69; C07C 29/14; C07C 29/172;**
C07B 2200/07; C07C 2602/42 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Studiengesellschaft Kohle gGmbH**
**45470 Mülheim (DE)**

(72) Inventors:
- **LIST, Benjamin**
  **45470 Mülheim an der Ruhr (DE)**
- **INIUTINA, Anna**
  **45470 Mülheim an der Ruhr (DE)**
- **GHOSH, Santanu**
  **West Bengal 721201 (IN)**
- **DE, Chandra Kanta**
  **45468 Mülheim (DE)**

# (54) PROCESS FOR PREPARING AN ENANTIOMERIC PURE DIELS-ALDER ADDUCT

(57) The present invention refers to a process for preparing an enantiomeric pure Diels-Alder Adduct, in particular a santalol derivative, i.e. (-)-(*Z*)-β-santalol.

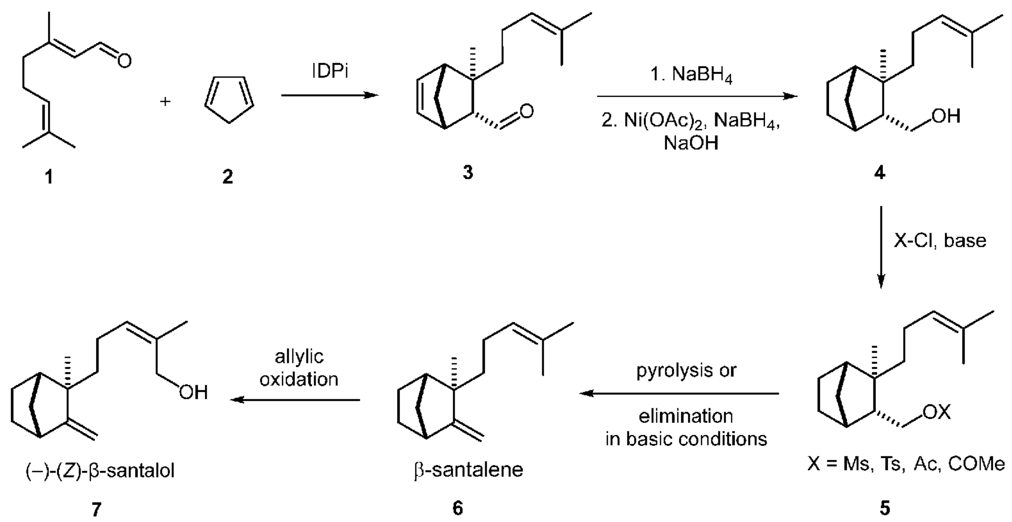


Figure 2

EP 4 678 622 A1

(52) Cooperative Patent Classification (CPC): (Cont.)

C-Sets
**C07C 29/14, C07C 33/14;**
**C07C 29/172, C07C 31/137;**
**C07C 45/69, C07C 47/445**

## Description

The present invention refers to a process for preparing an enantiomeric pure Diels-Alder adduct, in particular a santalol derivative, i.e. (-)-(*Z*)-β-santalol.

Sandalwood oil obtained from the trees of nowadays-protected species S. *album* is of a great demand for perfumery industry because of its distinctive soft, warm, creamy and woody scent. Chemical composition of sandalwood oil is mainly presented by two components: α- and β-santalols with last one being mainly responsible for the characteristic smell of sandalwood oil. It's worth mentioning that from four possible isomers of β-santalol only (-)-(*Z*)-β-santalol possess sandalwood odor while the opposite enantiomer is odorless and *E*-regioisomer is characterized by phenolic, medicinal odor.

Many attempts were made towards chemical synthesis of pure (-)-(*Z*)-β-santalol.Only two methods though were relevant for the industry. The approach reported by Fehr in Angewandte Chemie 48, 7221-7223 (2009) comprises 9 steps with the total yield of enantiopure (-)-(*Z*)-β-santalol 13.8% as disclosed in WO2009141781A.

A different approach reported by Chapuis as disclosed in WO2015067470A1 includes 8 steps with the total yield of (-)-(*Z*)-β-santalol 8%. Both routes as shown in Figure 1 require fine organic chemicals (not commercially available on large scales) and several chromatographic resolutions.

A more efficient approach for the assembling of (-)-(*Z*)-β-santalol backbone is an option via an enantioselective Diels-Alder reaction - as shown for the invention in Figure 2 - of geranial **1** and cyclopentadiene **2** - commercially available bulk chemicals and this strategy was basically realized by Weyerstahl in Liebigs Ann. Chem. 6, 1089-1099 (1981) albeit with very low yield (2%) and in non-asymmetric fashion leading to racemic mixtures.

In this inventive approach, all carbon atoms of the target molecule will be installed in one step together with both stereocenters, providing valuable intermediate **3**, which can be converted to **4** via simple carbonyl reduction and selective double bond hydrogenation. Alcohol elimination can be done via intermediating mesylate/tosylate or acetate **5**, but also another economically feasible option would be carbonate formation. The final regioselective allylic oxidation can be performed enzymatically or chemically.

In contrast, enzymatic approaches are less developed, but gaining a lot of attention recently. Most of them producing mixtures chemically resembling sandalwood oil rather than pure compounds or enriching natural products in β-santalol content.

Therefore, there is a need for an enantioselective and efficient route for production of (-)-(*Z*)-β-santalol which will be of a great importance for the industry.

The present inventors have considered to solve the problem of the invention by providing a more convenient, atom-economic and sustainable process for the synthesis of enantiopure (-)-(*Z*)-β-santalol.

The considerations of the inventors for the synthetic approach to (-)-(*Z*)-β-santalol are based on an enantioselective Diels-Alder reaction between an enal compound (1) and cyclopentadiene (2) as a first reaction step which reaction is represented in the following reaction scheme:

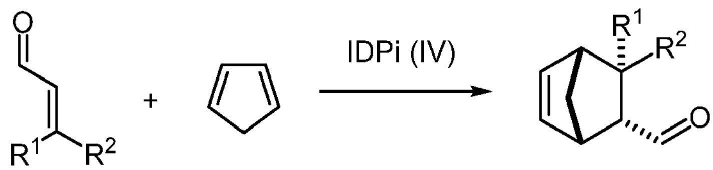


(1) (2) (3)

which reaction is catalyzed by a strong organic Br∅nsted acid imidophosphoroimidate (IDPi) as represented by general Formula (IV):

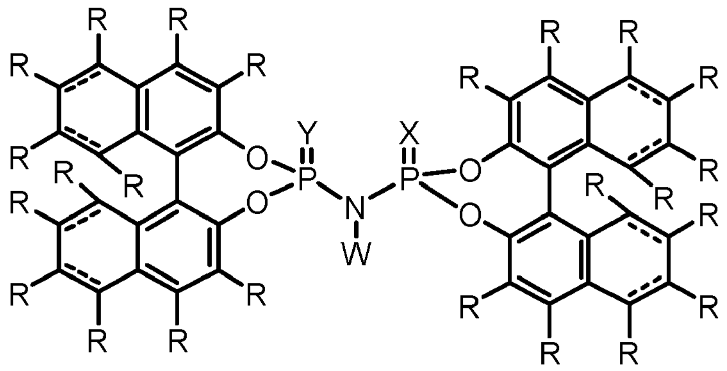

(IV)

wherein in said formula (IV):

R is the same or different on each position and is each selected from hydrogen, halogen, $SF_5$, $NO_2$, cyano, $C_1$ to $C_{20}$ straight chain, branched chain or cyclic aliphatic hydrocarbons, optionally having one or more halogens, preferably F or C!, $SF_5$, $NO_2$ or cyano on the aliphatic hydrocarbon, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or $C_5$ to $C_{18}$ heteroaromatic hydrocarbons optionally having one or more halogens, preferably F and/or Cl, $SF_5$, $NO_2$ or cyano on the aliphatic or aromatic hydrocarbon,
wherein any dashed line independently represents a double bond or two hydrogens,
wherein X and Y are the same or different and represent $NR^N$; wherein:
$R^N$ is an electron withdrawing group, being the same or different on each position and being selected from:

i. -alkyl, -CO-alkyl, -(CO)-O-alkyl, sulfinyl alkyl, sulfonyl alkyl, sulfonyl iminoalkyl, sulfonyl bisiminoalkyl, phosphinyl dialkyl, phosphonyl alkyl, alkyl phosphorane, N,N'-alkylimidazolidin-2-iminyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or Cl, cyano, nitro, or $SF_5$;
ii. -aryl, -CO-aryl, -(CO)-O-aryl, sulfinyl aryl, sulfonyl aryl, sulfonyl iminoaryl, sulfonyl iminosulfonylaryl, sulfonyl bisiminoaryl, phosphinyl diaryl, phosphinyl alkylaryl, phosphonyl aryl, aryl phosphoranes, aryl alkyl phosphoranes, N,N'-arylimidazolidin-2-iminyl, N-aryl-N'-alkylimidazolidin-2-iminyl, wherein aryl is a $C_6$ to $C_{18}$ aromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;
iii. -heteroaryl, -CO-heteroaryl, -(CO)-O-heteroaryl, sulfinyl heteroaryl, sulfonyl heteroaryl, -(P=O)-di-heteroaryl, phosphinyl diheteroaryl, phosphinyl aryl heteroaryl, phosphinyl heteroaryl alkyl, phosphonyl heteroaryl, heteroaryl phosphoranes, heteroaryl aryl phosphoranes, heteroaryl aryl alkyl phosphoranes, N,N'-heteroarylimidazolidin-2-iminyl, N-heteroaryl-N'-alkylimidazolidin-2-iminyl, N-heteroaryl-N'-arylimidazolidin-2-iminyl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;
and

wherein W is selected from hydrogen, halogen, a metal selected from Li, Na, K, Rb, Cs, Be, Mg, Ca, Sr, Ba Sc, Ti, V, Cr, Mn, Fe, Co, Ni, Cu, Zn, Y, Zr, Mo, Ru, Rh, Pd, Ag, Cd, W, Re, Os, Ir, Pt, Au, Hg, Al, Ga, In, Ge, Sn, Pb, As, Sb, Bi, Se, Te, La, Sm, Eu, Yb, U or a cationic organic group, a substituted borane $-BR^IR^{II}R^{III}$ or a substituted silicon $-SiR^IR^{II}R^{III}$, wherein $R^I$, $R^{II}$ and $R^{III}$ may be same or different and each stands for hydrogen, halogen, an optionally -O- bonded $C_1$ to $C_{20}$ straight chain, branched chain or cyclic aliphatic hydrocarbon, optionally having one or more unsaturated bonds or one or more hetero atoms in the chain, a $C_5$ to $C_{18}$ heteroaromatic hydrocarbon, a $C_6$ to $C_{18}$ aromatic hydrocarbon or partially arene-hydrogenated forms thereof, each hydrocarbon optionally being substituted by one or more groups selected from $C_1$ to $C_{20}$ straight chain, branched chain or cyclic aliphatic hydrocarbons, or one or more heterosubstituents, W being preferably selected from hydrogen, alkali metal or earth alkaline metal.

Thus, the present invention is directed in a first aspect to a process of an enantioselective Diels-Alder reaction between cyclopentadiene and an enal compound of Formula (1) as a first step which reaction is represented in the following reaction scheme:

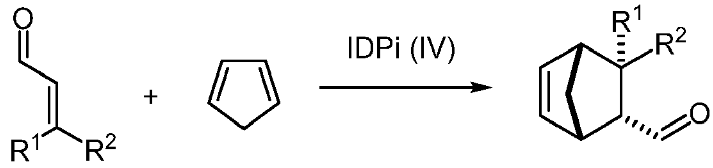


(1) (2) (3)

and which is catalyzed by a strong organic Brønsted acid imidophosphoroimidate (IDPi) as represented by general Formula (IV) as detailed before.

As substrates for the reaction, different exemplified compounds can be used such as an enal component of Formula (1) O=C-C=CR$^1$R$^2$ wherein $R^1$ represents $C_1$-$C_6$-alkyl, or -$C_1$-$C_6$-cycloalkyl, each alkyl optionally being substituted by a heteroatoms, $R^1$ being preferably methyl, and $R^2$ represents $C_1$-$C_{18}$-alkyl, $C_1$-$C_{18}$-cycloalkyl, $C_1$-$C_{18}$-alkenyl, $C_1$-$C_{12}$-alkynyl, $C_6$ to $C_{18}$ aromatic hydrocarbon or $C_5$ to $C_{18}$ heteroaromatic hydrocarbon, each alkyl, aromatic or heteroaromatic hydrocarbon optionally being substituted by one or more heterosubstituents, $C_1$ to $C_{20}$ straight chain, branched chain or cyclic aliphatic hydrocarbons, $C_2$ to $C_6$ alkenyl, $C_6$ to $C_{18}$ aromatic hydrocarbons or $C_5$ to $C_{18}$ heteroaromatic hydrocarbons, optionally having one or more heteroatoms on the aliphatic, aromatic or heteroaromatic hydrocarbon.

As enal compounds, terpene based enals may be used. When the enal of Formula (1) is geranial, the intermediate of Formula **3** for (-)-(*Z*)-β-santalol can be obtained.

As diene compound, simply cyclopentadiene or substituted forms thereof of Formula (2) wherein one or more hydrogens is replaced by $C_1$ to $C_6$ alkyl is used in the first step of the inventive synthesis.

In the first step of the inventive synthesis, the following reactions conditions preferably apply. The catalyst loading can be as low as 1.5 mol% (0.015 equiv. per 1 equiv. of geranial) with no limitation for further increasing.

The starting materials can be used in wide range of ratios, but the best results were obtained when 4 equivalents of cylcopentadiene were used in respect to geranial.

The reaction can be conducted in the presence of drying agents such as molecular sieves or inorganic salts ($Na_2SO_4$, $CaCl_2$, $MgSO_4$), but best results were obtained in the presence of molecular sieves 5Å or $CaSO_4$.

The drying reagents can be used in wide range of loading (180:1 to 600:1 [mg of drying agent: mmol of enal])), but best results were obtained with the ratio 500:1.

The described reaction proceeds in many organic solvents (for example, chlorinated solvents, ethers, hydrocarbons) at wide range of concentration (from 0.5 M up to 3 M [M=mol/L]), the best results in terms of yield and enantioselectivity, however, were obtained in MTBE with 1.7 M concentration of the starting materials.

The reaction proceeds in wide temperature interval (-80 - -10), however best results were obtained at -40. Correspondingly, different reaction times can be chosen depending on the used temperature (from 2 to 10 days), with the optimal reaction time 5 days at -40.

In another embodiment of the inventive process, geranial is used a starting material. Since said described process provides access to (-)-(*Z*)-β-santalol precursor **3** when geranial and cyclopendiene are used as substrates, the present invention also describes the use of this precursor for the synthesis of β-santalene (**6**).

As a next step on the way to β-santalene (**6**) from cycloadduct **3**, aldehyde reduction can be performed using a variety of hydride reducing agents, including but not limited to $NaBH_4$, $LiAlH_4$, $NaBH_3CN$, diisobutylaluminium hydride. $NaBH_4$ can be used as as one of the safest and most robust options.

The internal double bond in the compounds **3** can be selectively hydrogenated using the method described by Brown and Ahuja in Org. Chem. 35, 1900-1904 (1970).

When $NaBH_4$ used as a reducing agent for the discussed above steps, these steps can be performed in one-pot fashion.

The exo-double bond in β-santalene (**6**) has previously been reported to be formed via alcohol functionalization and subsequent elimination. For example, converting the alcohol moiety into an acetate, mesylate or tosylate can be performed, followed by an elimination under pyrolytic or basic conditions, correspondingly. However, the present inventors found all these conditions to be insufficient toward (-)-(*Z*)-β-santalol precursor **4.** Therefore, the present work also describes a novel approach for the formation of exo-double bond in (-)-(*Z*)-β-santalol precursors.

The approach of the inventors utilizes a transformation of the alcohol moiety into a carbonate using methyl chloroformate, and subsequent pyrolysis of the obtained carbonate. The formation of the carbonate allows obtaining an intermediate labile enough to undergo elimination under pyrolytic conditions, which was not possible in the case of acetate **5** (X=Ac). The advantages of this approach include: simple reaction set up, elimination reaction that does not require use of additional chemicals, simple non-toxic side products that do not require special handling ($CO_2$ and MeOH). Described sequence results in formation of β-santalene **6.**

The last step converts the unsaturated aliphatic side chain of β-santalene (**6**) into the desired allylic alcohol

containing fragment of (-)-(*Z*)-β-santalol.The discussed step has been extensively investigated in the prior art and can be performed enzymatically or chemically, resulting in the formation of (-)-(*Z*)-β-santalol.

In another embodiment of the inventive process, the catalyst is represented by the following formula (IVa) or (IVb) :

(IVa)

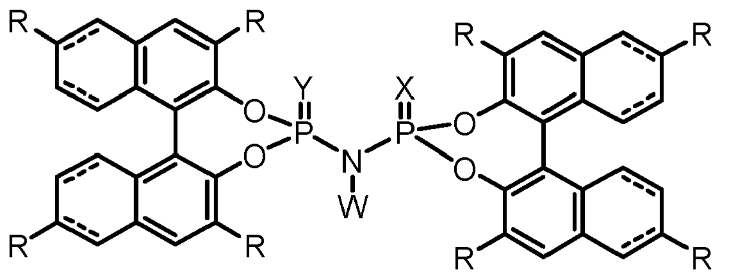

(IVb)

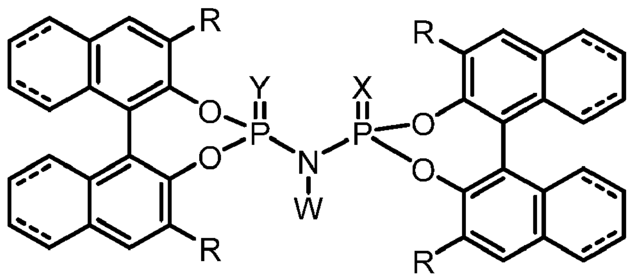

wherein the substituent R is the same or different on each position and is defined as in claim 1,
X and Y have the meanings as defined in claim 1,
any dashed line independently represents two hydrogens or preferably a double bond and W represents hydrogen, an alkali metal or an earth alkaline metal.

In another embodiment of the inventive process, the substituent R is the same or different on each position and represents halogen, a straight chain, branched chain or cyclic $C_1$ to $C_{20}$ aliphatic hydrocarbon or a $C_6$ to $C_{18}$ aromatic hydrocarbon, said aliphatic hydrocarbon and/or aromatic hydrocarbon being substituted by one or more halogens, preferably F and/or Cl, $SF_5$, $NO_2$ or a straight chain, branched chain or cyclic $C_1$ to $C_{20}$ aliphatic hydrocarbon, optionally being substituted by one or more halogens, preferably F and/or Cl, $SF_5$, $NO_2$ on the aliphatic hydrocarbon.

In a further embodiment of the inventive process as defined above for any formula (IV), (IVa) or (IVb), X and Y are the same or different and represent $NR^N$, wherein $R^N$ is an electron withdrawing group, being the same or different on each position and being selected from:

i. sulfinyl alkyl or sulfonyl alkyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or C!, cyano, nitro or $SF_5$;
ii. sulfinyl aryl, or sulfonyl aryl, wherein aryl is a $C_6$ to $C_{18}$ aromatic hydrocarbon, optionally having at least one substituent selected from $C_1$ to $C_6$ alkyl optionally substituted by at least one halogen , $C_1$ to $C_6$ alkoxy, halogen, preferably F and/or C!, cyano, nitro or $SF_5$;
iii. sulfinyl heteroaryl, or sulfonyl heteroaryl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or C!, cyano, nitro or $SF_5$; and

wherein R and W have the meanings as defined in any one of the preceding claims.

In yet another embodiment of the inventive process , the catalyst is represented by Formula (IVc)

(IVc)

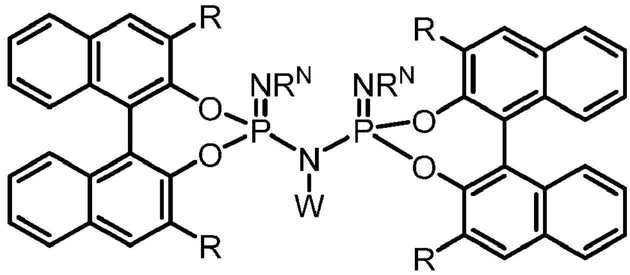

wherein R is the same on each position and is selected from hydrogen, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or $C_5$ to $C_{18}$ heteroaromatic hydrocarbons, optionally having one or more halogens, preferably F and/or Cl, $SF_5$, $NO_2$ or cyano on the aliphatic, aromatic or heteroaromatic hydrocarbon.

The catalyst as represented by formulae (IV), (IVa), (IVb) and (IVc) may be used as such in in the reaction system or in an immobilized form where the catalyst is bonded to a carrier or forms part thereof, prepared according to the process as described in EP23198382.6.

In the formulae (IV), (IVa), (IVb) and (IVc) , the broken/dashed line might optionally present a double bond, thus demonstrating a naphthalene ring system, or a hydrogenated double bond, demonstrating a 4H-naphthalene ring system, and both forms might be present in the catalysts as used in the inventive process.

The catalyst used here is based on imidodiphosphate (IDP) catalyst, the iminoimidodiphosphorimidate (iIDP) catalyst (List et al., J. Am. Chem. Soc. 2016, 138, 34, 10822) and imidodiphosphorimidate (IDPi) catalyst and may be prepared using the process as described in EP20200632.6. Used solvents are dried before use.

Definitions

The following definitions apply to the individual groups R, $R^N$ and W equally as follows.

A heteroatom or heterosubstituent as defined according to the invention can be selected from OH, F, Cl, Br, I, CN, $NO_2$, I-$R^S_2$, NO, NCO, -NCS, -SCN, $SO_3H$, a monohalogenomethyl group, a dihalogenomethyl group, a trihalogenomethyl group, $CF(CF3)_2$, $SF_5$, aliphatic, aromatic, heteroarmatic, primary, secondary, tertiary amine or ammonium bound through N atom, -O-alkyl (alkoxy), -O-aryl, -O-heteroaryl -O-Si$R^S_3$,-S-S-$R^S$, -S-$R^S$, -S(O)-$R^S$, -$S(O)_2$-$R^S$, -COOH, -$CO_2$-$R^S$, -B$R^S_2$, -P$R^S_2$, -OP$R^S_2$, amide, bound through C or N atom, formyl group, -C(O)-$R^S$, -COOM, where M is a metal such as Li, Na, K, Cs, Ag. $R^S$ may be, independently from each other, the same or different and is each an aliphatic, heteroaliphatic, aromatic or heteroaromatic group, each optionally being further substituted by one or more heterosubstituents, aliphatic, heteroaliphatic, aromatic or heteroaromatic groups; and/or optionally bridged by an -O- atom, represents a halogenide

Aliphatic hydrocarbons including alkyl, alkenyl and alkinyl and may comprise straight-chain, branched and cyclic hydrocarbons.

Heteroaliphatic is a hydrocarbon including alkyl, alkenyl and alkinyl which may comprise straight-chain, branched and cyclic hydrocarbons with one or more carbon atoms substituted with at least one heteroatom.

In more detail, $C_1$-$C_{20}$-alkyl can be straight chain or branched and has 1, 2, 3, 4, 5, 6, 7, 8, 9, 10, 11, 12, 13, 14, 15, 16, 17, 18, 19 or 20 carbon atoms. Alkyl might be $C_1$-$C_6$-alkyl, in particular methyl, ethyl, propyl, isopropyl, butyl, isobutyl, sec-butyl or tert-butyl, likewise pentyl, 1-, 2- or 3-methylpropyl, 1,1-, 1,2- or 2,2-dimethylpropyl, 1-ethylpropyl, hexyl, 1-, 2-, 3- or 4-methylpentyl, 1,1-, 1,2-, 1,3-, 2,2-, 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl. Substituted alkyl groups are trifluoromethyl, pentafluoroethyl and 1,1,1-trifluoroethyl.

Cycloalkyl might be cyclopropyl, cyclobutyl, cyclopentyl, cyclohexyl or cycloheptyl.

Alkenyl might be $C_2$-$C_{20}$ alkenyl. Alkinyl might be $C_2$-$C_{20}$ alkinyl.

Said unsaturated alkenyl- or alkinyl groups can be used for linking the inventive compounds to a carrier such as a polymer to serve for an immobilized catalyst.

Halogen such as F, Cl, Br or I.

Alkoxy is preferably $C_1$-$C_{10}$ alkoxy such as methoxy, ethoxy, propoxy, tert-butoxy, butoxy, pentoxy, hexyloxy, etc and isomers thereof.

$C_3$-$C_8$-Heterocycloalkyl having one or more heteroatoms selected from among N, O and S is preferably 2,3-dihydro-2-, -3-, -4- or -5-furyl, 2,5-dihydro-2-, -3-, -4- or -5-furyl, tetrahydro-2- or -3-furyl, 1,3-dioxolan-4-yl, tetrahydro-2- or -3-thienyl, 2,3-dihydro-1-, -2-, -3-, -4- or -5-pyrrolyl, 2,5-dihydro-1-, -2-, -3-, -4- or -5-pyrrolyl, 1-, 2- or 3-pyrrolidinyl, tetrahydro-1-, -2- or-4-imidazolyl, 2,3-dihydro-1-, -2-, -3-, -4- or-5-pyrazolyl, tetrahydro-1-, -3- or-4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-, -3- or -4-pyranyl, 1,4-dioxanyl, 1,3-dioxan-2-, -4- or -5-yl, hexahydro-1-, -3- or -4-pyridazinyl, hexahydro-1-, -2-, -4- or -5-pyrimidinyl, 1-, 2- or 3-piperazinyl, 1,2,3,4-tetrahydro-1-, -2-, -3-, -4-, -5-, -6-, -7- or -8-quinolyl, 1,2,3,4-tetrahydro-1-, -2-, -3-, -4-, -5-, -6-, -7- or -8-isoquinolyl, 2-, 3-, 5-, 6-, 7- or 8-3,4-dihydro-2H-benzo-1,4-oxazinyl.

Optionally substituted means unsubstituted or monosubstituted, disubstituted, trisubstituted, tetrasubstituted, pentasubstituted, or even further substituted for each hydrogen, such as persubstituted, on the hydrocarbon.

Aryl might be a $C_6$ to $C_{22}$ aromatic hydrocarbon and may be phenyl, naphthyl, anthracenyl, phenanthryl or biphenyl

Arylalkyl might be benzyl.

Heteroaryl may be a $C_5$ to $C_{18}$ heteroaromatic hydrocarbon and may have one or more heteroatoms selected from among N, O and S, and is preferably 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, also preferably 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or 4-pyridazinyl, pyrazinyl, 1-, 2-, 3-, 4-, 5-, 6- or 7-Indolyl, 4- or 5-isoindolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7- or 8-2H-benzo-1,4-oxazinyl, also preferably 1,3-benzodioxol-5-yl, 1,4-benzodioxan-6-yl, 2,1,3-benzothiadiazol-4- or -5-yl or 2,1,3-benzoxadiazol-5-yl.

The present invention is further illustrated by the attached Figures and Preparation Examples. In the Figures, it is shown in:

Figure 1: Routes to (-)-(*Z*)-β-santalol known in the prior art
Figure 2: Inventive route to (-)-(*Z*)-β-santalol

## Experimental Part

### Materials and Characterization

Unless otherwise stated, all reactions were magnetically stirred and conducted in oven-dried (90 °C) or flame-dried glassware in anhydrous solvents under argon, applying standard Schlenk techniques. Solvents and liquid reagents, as well as solutions of solid or liquid reagents were added via syringes, stainless steel or polyethylene cannulas through rubber septa or through a weak argon counter-flow. Solid reagents were added through a weak argon counter-flow. Cooling baths were prepared in Dewar vessels, filled with ice/water (0 °C), cooled acetone (< -78 °C) or dry ice/acetone (-78 °C). Heated oil baths were used for reactions requiring elevated temperatures. Solvents were removed under reduced pressure at 40 °C using a rotary evaporator, and unless otherwise stated, the remaining compound was dried in high vacuum ($10^{-3}$ mbar) at rt. All given yields are isolated yields of chromatographically and NMR-spectroscopically pure materials, unless otherwise stated.

Chemicals were purchased from commercial suppliers (including abcr, Acros, Alfa Aesar, Fluorochem, Sigma-Aldrich, and TCI) and used without further purification unless otherwise stated.

Solvents were dried by distillation from an appropriate drying agent in the technical department of the Max-Planck-Institut für Kohlenforschung and received in Schlenk flasks under argon. Other anhydrous solvents were purchased from commercial suppliers and used as received.

Reactions were monitored by thin layer chromatography (TLC) on silica gel pre-coated plastic sheets (0.2 mm, Macherey-Nagel). Visualization was accomplished by irradiation with UV light (254 nm and 366 nm) and/or p-anisaldehyd e stain.

Column chromatography was carried out using Merck silica gel (60 Å, 230-400 mesh, particle size 0.040-0.063 mm) using technical grade solvents. Elution was accelerated using compressed air. All fractions containing a desired substance were combined and concentrated in vacuo, then redissolved in an appropriate solvent and filtered through cotton to remove silica residues.

$^{1}H$, $^{13}C$, $^{19}F$, $^{31}P$ nuclear magnetic resonance (NMR) spectra were recorded on a Bruker AV-500, AV-400 or DPX-300 spectrometer in a suitable deuterated solvent. The solvent employed and respective measuring frequency are indicated for each experiment. Chemical shifts are reported with $Me_4Si$ serving as a universal reference of all nuclides and with two or one digits after the comma. The resonance multiplicity is described as s (singlet), d (doublet), t (triplet), q (quadruplet), p (pentet), hept (heptet), m (multiplet), and b (broad). All spectra were recorded at 298 K unless otherwise noted, processed with the program MestReNova 14.3, and coupling constants are reported as observed. The residual deuterated solvent signal relative to $Me_4Si$ was used as the internal reference in $^{1}H$ NMR spectra (e.g. $CDCl_3$ = 7.26 ppm)

and are reported as follows: chemical shift in ppm (multiplicity, coupling constant J in Hz, number of protons). $^{13}C$, $^{19}F$, $^{31}P$ NMR spectra were referenced according to Ξ-vaiues (IUPAC recommendations 2008)[2] relative to the internal references set in $^{1}H$ NMR spectra (e.g. $^{13}C$: $Me_4Si$, $^{19}F$: $CCl_3F$, $^{31}P$: $H_3PO_4$; each 0.00 ppm). All spectra are broadband decoupled unless otherwise noted.

Specific rotations $[\alpha]_T^D$ were measured with a Rudolph RA Autopol IV Automatic Polarimeter at the indicated temperature (T) with a sodium lamp (sodium D line, $\lambda$ = 589 nm). Measurements were performed in an acid resistant 1 mL cell (50 mm length) with concentrations (g/(100 mL)) reported in the corresponding solvent.

EXAMPLES

General Procedure for the Preparation of Cvcloadduct 3 [Diels-Alder Reaction]:

Activated molecular sieves 5Å (35 g) and IDPi catalyst [Ar = p-tBu$C_6H_4$, X = $C_6F_5$] (1.5 mol%, 1.48 mmol, 2.47 g) were added in a flame-dried reaction vessel together with a stirring bar. MTBE (1.8M, 60 mL) was added and the obtained mixture was stirred for 15 min at room temperature until the catalyst is fully dissolved. Then geranial (1 equiv., 100 mmol, 17.05 mL) was added and the reaction mixture was stirred for additional 15 min before being cooled down in dry ice bath. To the cool reaction mixture cyclopentadiene was added (4 equiv., 400 mmol, 33.05 mL) and the reaction vessel was put into -40°C cryostat for 5 days. After completion of the reaction (tracked by NMR) it was quenched by the addition of TEA (0.03 equiv., 0.4 mL) and allowed to warm up to room temperature. Molecular sieves were removed by filtration through a fritted glass filter and obtained solution was concentrated under reduced pressure.

The crude product was purified by column chromatography hexane/MTBE (6:1) to afford the pure product in 76.5% (16.7g) yield, 98% ee as yellowish oil.

The catalyst was recovered by washing the chromatography column with hexane/MTBE (1:1) and acidified over Dowex resin.

$^{1}H$ NMR (501 MHz, $CDCl_3$): mixture of two diastereomers -10:1, NMR data for the major diastereomer is reported. δ 9.36 (d, *J* = 4.0 Hz, 1H), 6.36 (dd, J = 5.7, 2.8 Hz, 1H), 6.31 (dd, *J* = 5.8, 3.2 Hz, 1H), , 5.13 (tp, *J* = 7.1, 1.4 Hz, 1H), 3.02 (t, *J* = 2.9 Hz, 1H), 2.57 (p, *J* = 1.7 Hz, 1H), 2.52 (t, *J* = 3.7 Hz, 1H), 2.12 - 2.04 (m, 2H), 1.75 - 1.59 (m, 10H), 0.93 (s, 3H).

**$^{13}C$ NMR** (126 MHz, $CDCl_3$) δ 206.71, 138.25, 134.14, 131.61, 124.35, 61.65, 52.67, 48.80, 47.17, 45.88, 44.43, 26.91, 25.67, 23.68, 20.46.

**$[\alpha]_D^{25}$** = - 66.55 (c = 0.57, $CHCl_3$)

General Procedure for Aldehyde Reduction with $NaBH_4$:

To a stirred solution of aldehyde obtained in previous step (1.0 equiv., 6.89 mmol, 1505 mg) in EtOH (0.17M, 40 mL) was added $NaBH_4$ (1.5 equiv., 10.34 mmol, 391 mg) at room temperature and stirring was continued for 3 h. The reaction mixture was diluted with $Et_2O$ (40 mL), washed with $H_2O$ (3x100 mL) and saturated aqueous NaCl (100 mL), dried over anhydrous $MgSO_4$ and concentrated under reduced pressure to give crude alcohol in 84% yield (1280 mg) as colorless oil. The crude product was used directly in the next step. Analytical sample was purified by column chromatography hexane/MTBE (4:1) to afford the pure product in 80% yield.

**$^{1}H$ NMR** (501 MHz, $CDCl_3$) mixture of two diastereomers 10:1, NMR data for the major diastereomer is reported. δ 6.23 (dd, J = 5.7, 3.1 Hz, 1H), 6.18 - 6.13 (m, 1H), 5.13 (tdd, J = 7.2, 2.9, 1.5 Hz, 1H), 3.45 (dd, *J* = 10.5, 6.1 Hz, 1H), 3.27 - 3.19 (m, 2H), 2.90 (d, *J* = 3.7 Hz, 1H), 2.45 (q, *J* = 1.7 Hz, 1H), 2.04 (dq, *J* = 12.7, 6.7 Hz, 2H), 1.70 (d, *J* = 1.6 Hz, 3H), 1.69 - 1.60 (m, 5H), 1.56 (ddd, *J* = 13.4, 11.1, 5.7 Hz, 1H), 1.51 - 1.38 (m, 2H), 0.75 (s, 3H).

**$^{13}C$ NMR** (126 MHz, $CDCl_3$) δ 138.4, 133.8, 131.2, 125.0, 64.6, 53.1, 51.3, 46.6, 45.4, 44.7, 43.7, 25.7, 23.9, 18.8, 17.6.

**$[\alpha]_D^{25}$** = - 17.59 (c = 0.71, $CHCl_3$)

Reduction of the Double Bond with Ni P2 to Afford Compound **4:**

To a solution of $Ni(OAc)_2 \times 4H_2O$ (0.2 equiv., 240 mg, 0.96 mmol) in 95% ethanol (100 mL) was added in one portion a solution of $NaBH_4$ (0.16 equiv., 29 mg, 0.77 mmol) and NaOH (0.008 equiv., 1.5 mg, 0.038 mmol) in aqueous ethanol (35 mL EtOH + 0.5 mL of $H_2O$). The reaction flask was purged with hydrogen and the substrate unsaturated alcohol (1 equiv., 1050 mg, 4.8 mmol) was added as a solution in 20 mL of EtOH. Reaction mixture was stirred at room temperature for 3 h under $H_2$ atmosphere.

The reaction mixture was then poured into 1M HCl (150 mL) and the mixture was extracted with diethyl ether (2x100 mL). The organic phases were washed with brine, dried over $MgSO_4$ and evaporated to afford the product as yellow

oil in 82% yield, which was used without purification in the next step.

**$^1$H NMR** (501 MHz, $CDCl_3$) mixture of two diastereomers -10:1, NMR data for the major diastereomer is reported. δ 5.10 (dddd, *J* = 8.5, 5.7, 2.9, 1.4 Hz, 1H), 3.74 (q, *J* = 7.0 Hz, 1H), 3.66 - 3.60 (m, 2H), 2.30 (t, *J* = 3.2 Hz, 1H), 2.01 - 1.89 (m, 3H), 1.71 - 1.56 (m, 10H), 1.34 - 1.21 (m, 5H), 0.84 (s, 3H).

**$^{13}$C** NMR (126 MHz, $CDCl_3$) δ 131.09, 125.04, 61.32, 51.65, 47.24, 44.65, 39.92, 39.34, 37.01, 25.69, 24.57, 22.87, 20.65, 17.60, 17.02.

**$[\alpha]_D^{25}$** = - 3.55 (c = 0.56, $CHCl_3$)

Two discussed above steps can also be performed in a one-pot fashion starting with reduction of double bond and then adding excess of $NaBH_4$ (2 equiv.) to provide product **4** in 62% overall yield.

Synthesis of Carbonate **5** (X = COMe):

To a solution of the alcohol (1 equiv., 714 mg, 3.21 mmol) in THF (40 ml) cooled to -15°C was added 2.5M BuLi in hexanes (1.05 equiv., 1.35 mL, 3.37 mmol). After complete addition, the solution was stirred for 15 min and then treated with methyl chloroformate (1.05 equiv., 0.266 mL, 3.37 mmol). Stirring was continued at 0°C for 20 min. After addition of $Et_2O$ (30 mL) and $H_2O$ (40 mL), organic layer was separated and the aqueous layer was extracted with ether (40 mL). Combined organic layers were washed with $H_2O$ (50x3 mL), then sat. aq. NaCl (50 mL), dried over $Na_2SO_4$, and evaporated to afford the crude carbonate which was purified by column chromatography (hexane:MTBE 10:1) to afford the pure product in 85% yield.

**$^1$H NMR** (501 MHz, $CDCl_3$) mixture of two diastereomers 10:1, NMR data for the major diastereomer is reported. δ 5.10 (tdp, *J* = 6.9, 4.2, 1.4 Hz, 1H), 4.19 - 4.13 (m, 2H), 3.79 (s, 3H), 2.27 (td, *J* = 3.9, 2.0 Hz, 1H), 2.01 - 1.90 (m, 3H), 1.83 - 1.75 (m, 1H), 1.72 - 1.55 (m, 8H), 1.47 - 1.18 (m, 6H), 0.87 (s, 3H).

**$^{13}$C NMR** (126 MHz, $CDCl_3$) δ 155.91, 131.15, 124.96, 67.24, 54.61, 47.73, 47.52, 47.22, 44.38, 39.82, 37.04, 25.68, 24.50, 22.79, 20.81, 17.58, 17.16.

**$[a]_D^{25}$** = - 14.36 (c = 0.59, $CHCl_3$)

Pyrolysis of the Carbonate to Yield Product **6** (β-santalene):

Carbonate obtained in previous step (288 mg, 1 mmol) was pyrolyzed at 580 °C (10 ml/min, quartz tube, $N_2$). Obtained oil was purified column chromatography hexane/MTBE (9:1) to afford the pure product in 64% (135 mg) yield as yellowish oil.

**$^1$H NMR** (501 MHz, $CDCl_3$) δ 5.19 - 5.04 (m, 1H), 4.77 (s, 1H), 4.51 (s, 1H), 2.69 (dd, *J* = 4.7, 1.7 Hz, 1H), 2.18 - 1.85 (m, 3H), 1.72 (s, 3H), 1.65 (m, 3H), 1.59 - 1.01 (m, 8H), 1.03 (s, 3H).

**$^{13}$C NMR** (126 MHz, $CDCl_3$) δ 166.4, 130.9, 124.9, 99.2, 46.8, 44.8, 44.4, 41.2, 37.0, 29.6, 25.5, 23.7, 23.5, 22.7, 17.4.

**$[a]_D^{25}$**= - 107.16 (c = 0.69, $CHCl_3$)

β-santalene can be converted to β-santalol via one of the following procedures: as disclosed in WO2015040197A1 (enzymatic approach) or in WO2021063831A1 (chemical approach).

## Claims

1. Process of an enantioselective Diels-Alder reaction whereby an enal compound of Formula (1) O=C-C=$CR^1R^2$ is reacted with cyclopentadiene of Formula (2) whereby a cycloaddition compound (3) is obtained which reaction is represented in the following reaction scheme:

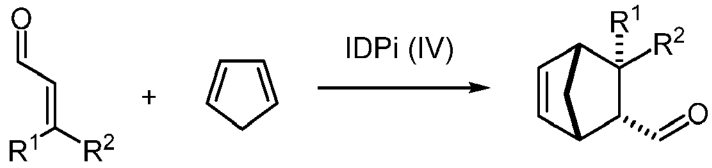


(1) (2) (3)

Wherein:

$R^1$ represents $C_1$-$C_6$-alkyl, or -$C_1$-$C_6$-cycloalkyl, each alkyl optionally being substituted by one or more heteroa-

toms, $R^1$ preferably being methyl,
$R^2$ represents $C_1$-$C_{18}$-alkyl, $C_1$-$C_{18}$-cycloalkyl, $C_1$-$C_{18}$-alkenyl, $C_1$-$C_{12}$-alkynyl, $C_6$ to $C_{18}$ aromatic hydrocarbon or $C_5$ to $C_{18}$ heteroaromatic hydrocarbon, each alkyl, aromatic or heteroaromatic hydrocarbon optionally being substituted by one or more heterosubstituents, $C_1$ to $C_{20}$ straight chain, branched chain or cyclic aliphatic hydrocarbons, $C_2$ to $C_6$ alkenyl, $C_6$ to $C_{18}$ aromatic hydrocarbons or $C_5$ to $C_{18}$ heteroaromatic hydrocarbons, optionally having one or more heteroatoms on the aliphatic, aromatic or heteroaromatic hydrocarbon, and wherein the IDPi-catalyst is represented by Formula (IV)

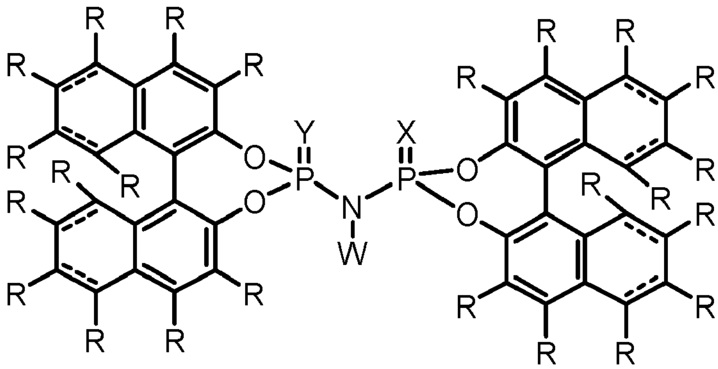

(IV)

wherein in said formula (IV):

R is the same or different on each position and is each selected from hydrogen, halogen, $SF_5$, $NO_2$, cyano, $C_1$ to $C_{20}$ straight chain, branched chain or cyclic aliphatic hydrocarbons, optionally having one or more halogens, preferably F or Cl, $SF_5$, $NO_2$ or cyano on the aliphatic hydrocarbon, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or $C_5$ to $C_{18}$ heteroaromatic hydrocarbons optionally having one or more heteroatoms, preferably F and/or Cl, $SF_5$, $NO_2$ or cyano on the aliphatic or aromatic hydrocarbon,
wherein any dashed line independently represents a double bond or two hydrogens,
wherein X and Y are the same or different and represent $NR^N$; wherein:
$R^N$ is an electron withdrawing group, being the same or different on each position and being selected from:

i. -alkyl, -CO-alkyl, -(CO)-O-alkyl, sulfinyl alkyl, sulfonyl alkyl, sulfonyl iminoalkyl, sulfonyl bisiminoalkyl, phosphinyl dialkyl, phosphonyl alkyl, alkyl phosphorane, N,N'-alkylimidazolidin-2-iminyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or Cl, cyano, nitro, or $SF_5$;
ii. -aryl, -CO-aryl, -(CO)-O-aryl, sulfinyl aryl, sulfonyl aryl, sulfonyl iminoaryl, sulfonyl iminosulfonylaryl, sulfonyl bisiminoaryl, phosphinyl diaryl, phosphinyl alkylaryl, phosphonyl aryl, aryl phosphoranes, aryl alkyl phosphoranes, N,N'-arylimidazolidin-2-iminyl, N-aryl-N'-alkylimidazolidin-2-iminyl, wherein aryl is a $C_6$ to $C_{18}$ aromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;
iii. -heteroaryl, -CO-heteroaryl, -(CO)-O-heteroaryl, sulfinyl heteroaryl, sulfonyl heteroaryl, -(P=O)-diheteroaryl, phosphinyl diheteroaryl, phosphinyl arylheteroaryl, phosphinyl heteroaryl alkyl, phosphonyl heteroaryl, heteroaryl phosphoranes, heteroaryl aryl phosphoranes, heteroaryl aryl alkyl phosphoranes, N,N'-heteroarylimidazolidin-2-iminyl, N-heteroaryl-N'-alkylimidazolidin-2-iminyl, N-heteroaryl-N'-arylimidazolidin-2-iminyl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;

and
wherein W is selected from hydrogen, halogen, a metal selected from Li, Na, K, Rb, Cs, Be, Mg, Ca, Sr, Ba Sc, Ti, V, Cr, Mn, Fe, Co, Ni, Cu, Zn, Y, Zr, Mo, Ru, Rh, Pd, Ag, Cd, W, Re, Os, Ir, Pt, Au, Hg, Al, Ga, In, Ge, Sn, Pb, As, Sb, Bi, Se, Te, La, Sm, Eu, Yb, U or a cationic organic group, a substituted borane $-BR^IR^{II}R^{III}$ or a substituted silicon $-SiR^IR^{II}R^{III}$, wherein $R^I$, $R^{II}$ and $R^{III}$ may be same or different and each stands for hydrogen,

halogen, an optionally -O- bonded $C_1$ to $C_{20}$ straight chain, branched chain or cyclic aliphatic hydrocarbon, optionally having one or more unsaturated bonds or one or more hetero atoms in the chain, a $C_5$ to $C_{18}$ heteroaromatic hydrocarbon, a $C_6$ to $C_{18}$ aromatic hydrocarbon or partially arene-hydrogenated forms thereof, each hydrocarbon optionally being substituted by one or more groups selected from $C_1$ to $C_{20}$ straight chain, branched chain or cyclic aliphatic hydrocarbons, or one or more heterosubstituents, W being preferably selected from hydrogen, alkali metal or earth alkaline metal.

**2.** Process according to claim 1, wherein the catalyst is represented by the following formula (IVa) or (IVb) :

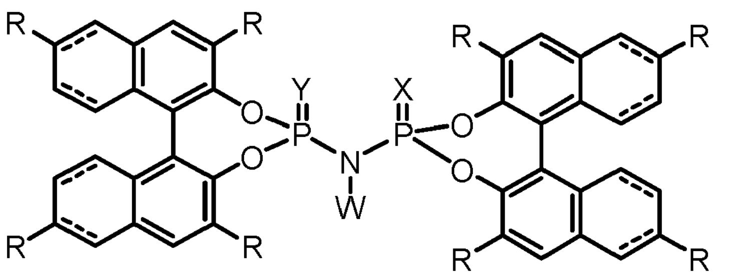

(IVa)

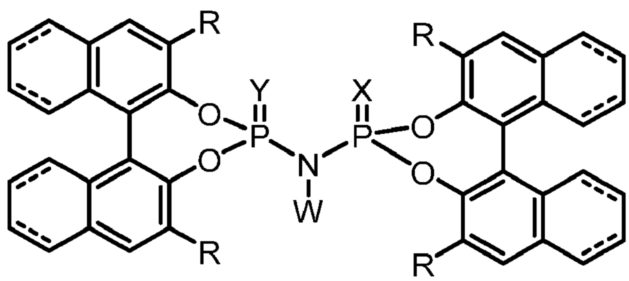

(IVb)

wherein the substituent R is the same or different on each position and is defined as in claim 1,
X and Y have the meanings as defined in claim 1,
any dashed line independently represents two hydrogens or preferably a double bond and W represents hydrogen, an alkali metal or an earth alkaline metal.

**3.** Process according to any one of claims 1 to 2, wherein the substituent R is the same or different on each position and represents halogen, a straight chain, branched chain or cyclic $C_1$ to $C_{20}$ aliphatic hydrocarbon or a $C_6$ to $C_{18}$ aromatic hydrocarbon, said aliphatic hydrocarbon and/or aromatic hydrocarbon being substituted by one or more halogens, preferably F and/or Cl, $SF_5$, $NO_2$ or a straight chain, branched chain or cyclic $C_1$ to $C_{20}$ aliphatic hydrocarbon, optionally being substituted by one or more halogens, preferably F and/or Cl, $SF_5$, $NO_2$ on the aliphatic hydrocarbon.

**4.** Process according to any one of the preceding claims, wherein, in any formula (IV), (IVa) or (IVb), X and Y are the same or different and represent $NR^N$, wherein $R^N$ is an electron withdrawing group, being the same or different on each position and being selected from:

j. sulfinyl alkyl or sulfonyl alkyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or C!, cyano, nitro or $SF_5$;
ii. sulfinyl aryl, or sulfonyl aryl, wherein aryl is a $C_6$ to $C_{18}$ aromatic hydrocarbon, optionally having at least one substituent selected from $C_1$ to $C_6$ alkyl optionally substituted by at least one halogen , $C_1$ to $C_6$ alkoxy, halogen, preferably F and/or C!, cyano, nitro or $SF_5$;
iii. sulfinyl heteroaryl, or sulfonyl heteroaryl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or C!, cyano, nitro or $SF_5$; and

wherein R and W have the meanings as defined in any one of the preceding claims.

**5.** Process according to any one of the preceding claims, wherein the catalyst is represented by Formula (IVc)

(IVc)

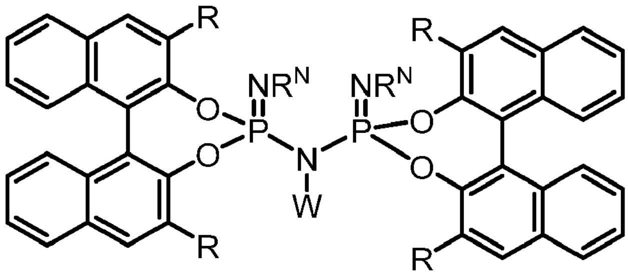

wherein R is the same on each position and is selected from hydrogen, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or $C_5$ to $C_{18}$ heteroaromatic hydrocarbons, optionally having one or more halogens, preferably F and/or Cl, $SF_5$, $NO_2$ or cyano on the aliphatic, aromatic or heteroaromatic hydrocarbon.

**6.** Process according to any one of the preceding claims wherein geranial is used as enal compound (1) which is reacted with cycylopentadiene of Formula (2) whereby an enantiopure Diels-Alder cycloaddition adduct of Formula (3) is obtained.

**7.** Process according to claim 6, comprising the additional steps of converting the enantiopure Diels-Alder cycloaddition adduct of Formula (3) via a carbonyl reduction and selective double bond hydrogenation into compound 4, eliminating the hydroxyl group of compound 4 preferably via intermediating mesylate/tosylate/acetate or carbonate to compound 6, and followed by a final regioselective allylic oxidation performed enzymatically or chemically whereby enantiopure (-)-(*Z*)-β-santalol is obtained.

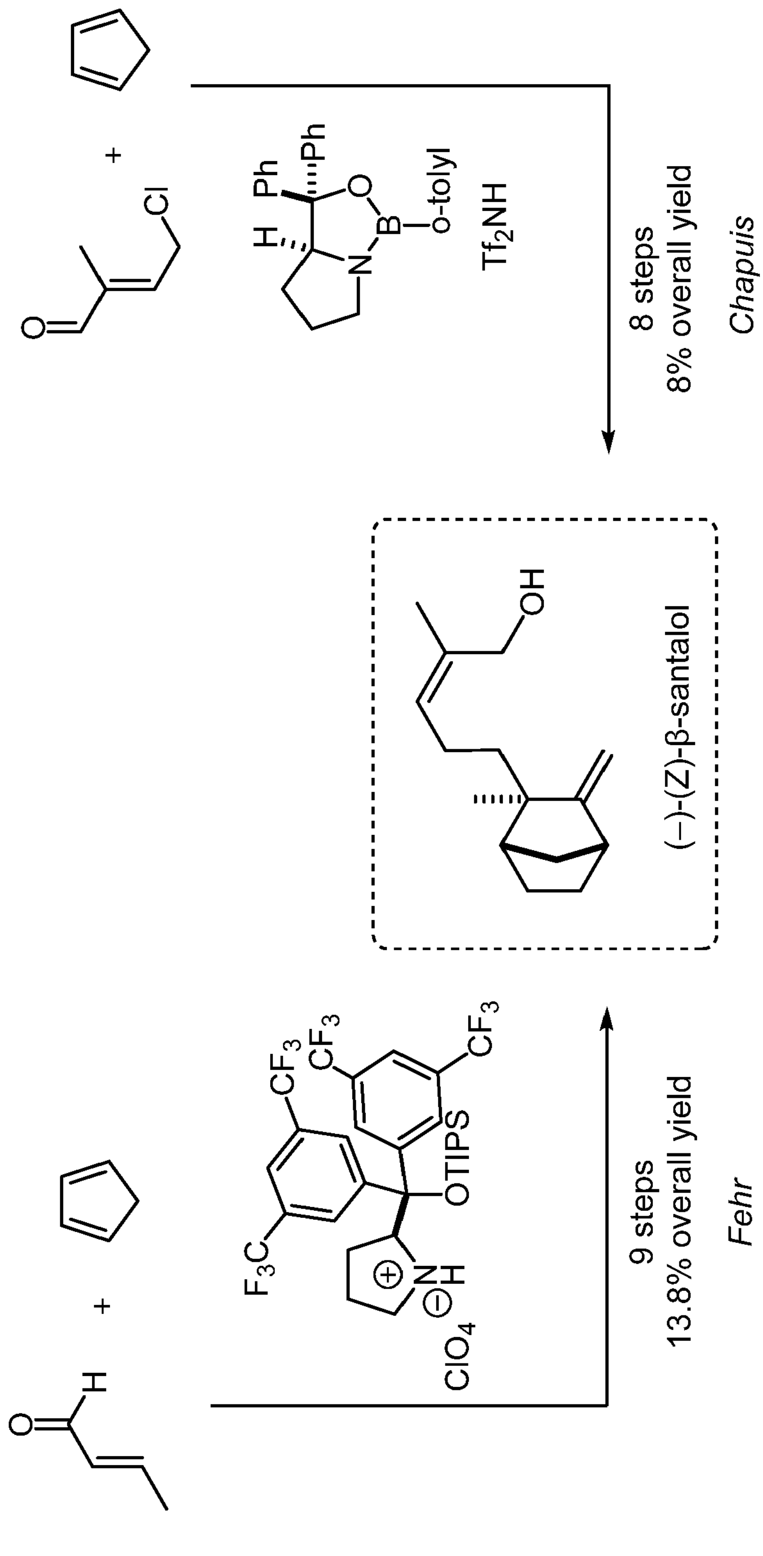

Tf$_2$NH
8 steps
8% overall yield
Chapuis
(−)-(Z)-β-santalol
9 steps
13.8% overall yield
Fehr


Figure 1

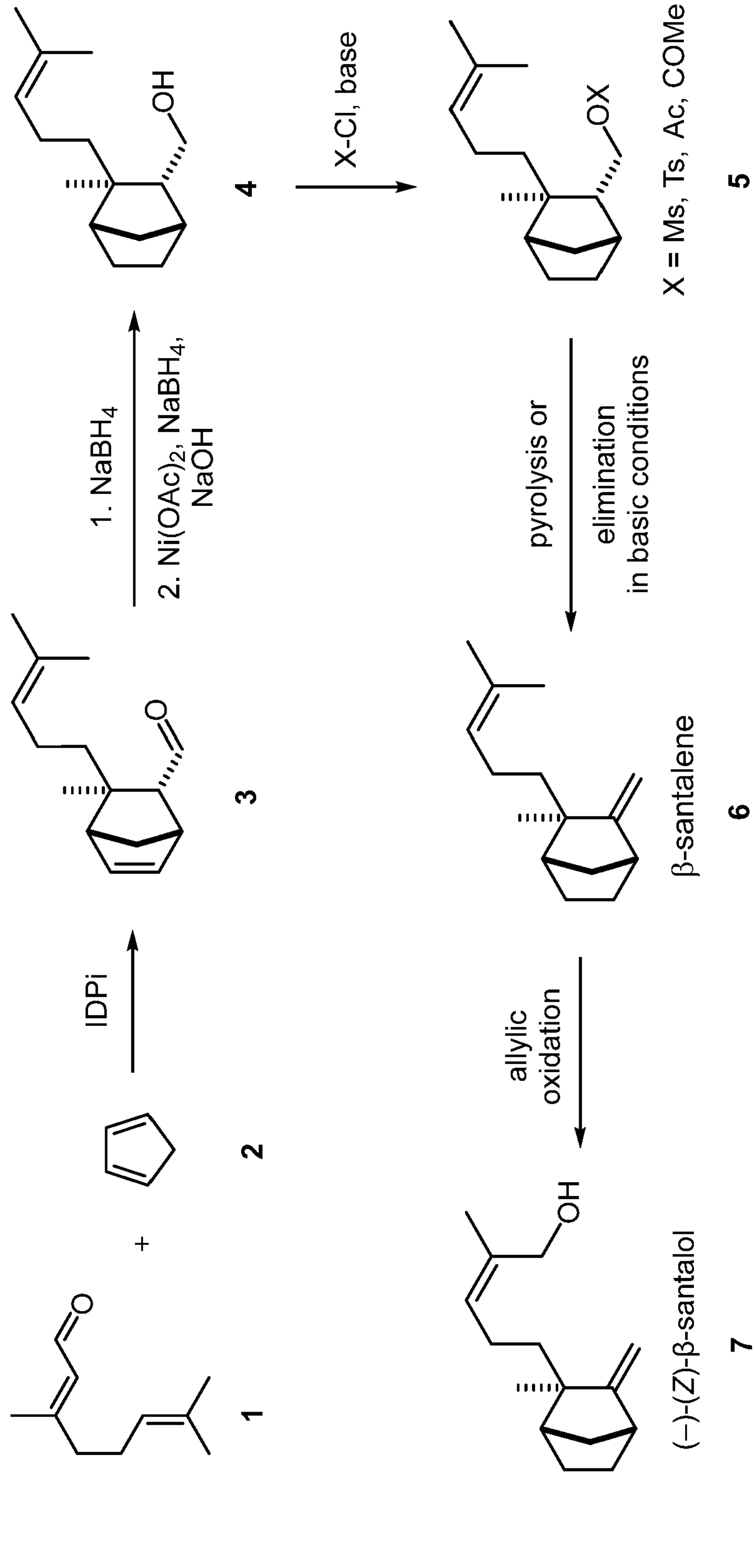


1

+

2

3

4

X = Ms, Ts, Ac, COMe

5

β-santalene

6

(−)-(Z)-β-santalol

7

Figure 2

Europäisches Patentamt
European Patent Office
Office européen des brevets

# EUROPEAN SEARCH REPORT

Application Number
EP 24 18 6979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YUJIRO HAYASHI ET AL: "Asymmetric Diels-Alder Reaction of [alpha]-Substituted and [beta],[beta]-Disubstituted [alpha],[beta]-Enals via Diarylprolinol Silyl Ether for the Construction of All-Carbon Quaternary Stereocenters", CHEMISTRY - A EUROPEAN JOURNAL, JOHN WILEY & SONS, INC, DE, vol. 22, no. 44, 7 September 2016 (2016-09-07), pages 15874-15880, XP071879533, ISSN: 0947-6539, DOI: 10.1002/CHEM.201602345 * table 4 * ----- | 1-7 | INV. C07C29/14 C07C29/17 C07C45/69 C07C33/14 C07C31/137 C07C47/445 |
| Y | KIM HYEJIN ET AL: "A multi-substrate screening approach for the identification of a broadly applicable Diels-Alder catalyst", NATURE COMMUNICATIONS, vol. 10, no. 1, 15 February 2019 (2019-02-15), pages 1-6, XP093242288, UK ISSN: 2041-1723, DOI: 10.1038/s41467-019-08374-z Retrieved from the Internet: URL:https://www.nature.com/articles/s41467-019-08374-z> * table 1 * ----- | 1-7 | TECHNICAL FIELDS SEARCHED (IPC) C07C |
| A | WO 2017/037141 A1 (STUDIENGESELLSCHAFT KOHLE MBH [DE]) 9 March 2017 (2017-03-09) * claims, examples * ----- -/-- | 1-7 | |

The present search report has been drawn up for all claims

2

| Place of search | Date of completion of the search | Examiner |
|---|---|---|
| The Hague | 4 February 2025 | Karolak, Ewelina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 18 6979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SANTANU GHOSH ET AL: "Strong and Confined Acids Control Five Stereogenic Centers in Catalytic Asymmetric Diels-Alder Reactions of Cyclohexadienones with Cyclopentadiene", ANGEWANDTE CHEMIE INTERNATIONAL EDITION, VERLAG CHEMIE, HOBOKEN, USA, vol. 59, no. 30, 11 March 2020 (2020-03-11), pages 12347-12351, XP072094642, ISSN: 1433-7851, DOI: 10.1002/ANIE.202000307 * table 1 * ----- | 1-7 | |
| | | | TECHNICAL FIELDS SEARCHED (IPC) |

The present search report has been drawn up for all claims

| Place of search | Date of completion of the search | Examiner |
|---|---|---|
| The Hague | 4 February 2025 | Karolak, Ewelina |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 24 18 6979

This annex lists the patent family members relating to the patent documents cited in the above-mentioned European search report. The members are as contained in the European Patent Office EDP file on
The European Patent Office is in no way liable for these particulars which are merely given for the purpose of information.

04-02-2025

| Patent document cited in search report | Publication date | Patent family member(s) | Publication date |
|---|---|---|---|
| WO 2017037141 A1 | 09-03-2017 | AU 2016316712 A1 | 08-03-2018 |
| | | BR 112018002669 A2 | 02-10-2018 |
| | | CA 2994788 A1 | 09-03-2017 |
| | | CN 107922449 A | 17-04-2018 |
| | | CN 117659092 A | 08-03-2024 |
| | | EA 201890632 A1 | 28-09-2018 |
| | | EP 3138845 A1 | 08-03-2017 |
| | | EP 3344639 A1 | 11-07-2018 |
| | | ES 2814328 T3 | 26-03-2021 |
| | | IL 257283 A | 29-03-2018 |
| | | JP 7010822 B2 | 10-02-2022 |
| | | JP 2018534341 A | 22-11-2018 |
| | | KR 20180044420 A | 02-05-2018 |
| | | US 2018339999 A1 | 29-11-2018 |
| | | WO 2017037141 A1 | 09-03-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009141781 A
- WO 2015067470 A1
- EP 23198382
- EP 20200632
- WO 2015040197 A1
- WO 2021063831 A1


### Non-patent literature cited in the description


- *Angewandte Chemie*, 2009, vol. 48, 7221-7223
- **WEYERSTAHL**. *Liebigs Ann. Chem.*, 1981, vol. 6, 1089-1099
- **BROWN** ; **AHUJA**. *Org. Chem.*, 1970, vol. 35, 1900-1904
- **LIST et al.** *J. Am. Chem. Soc.*, 2016, vol. 138 (34), 10822